**Europäisches Patentamt**

**European Patent Office**

**Office européen des brevets**

(19)

(11) Veröffentlichungsnummer: **0 017 162**
**B1**

(12) # EUROPÄISCHE PATENTSCHRIFT

(45) Veröffentlichungstag der Patentschrift:
**08.06.83**

(51) Int. Cl.³: **A 61 B 5/08**

(21) Anmeldenummer: **80101609.8**

(22) Anmeldetag: **26.03.80**

(54) **Gerät zur Lungenfunktionsanalyse.**

(30) Priorität: **29.03.79 DE 2912391**

(43) Veröffentlichungstag der Anmeldung:
**15.10.80 Patentblatt 80/21**

(45) Bekanntmachung des Hinweises auf die Patenterteilung:
**08.06.83 Patentblatt 83/23**

(84) Benannte Vertragsstaaten:
**AT CH FR GB NL SE**

(56) Entgegenhaltungen:
**DE-B-1 918 566**
**DE-B-2 541 691**
**US-A-3 527 205**
**US-A-3 659 590**

(73) Patentinhaber: **SIEMENS AKTIENGESELLSCHAFT,**
**Berlin und München Wittelsbacherplatz 2,**
**D-8000 München 2 (DE)**

(72) Erfinder: **Volker, Korn, Dipl.-Ing., Herrnhüttestrasse 10,**
**D-8500 Nürnberg (DE)**

ACTORUM AG

## Gerät zur Lungenfunktionsanalyse

Die Erfindung bezieht sich auf ein Gerät zur Lungenfunktionsanalyse, insbesondere zur Messung von funktionellen Atemvolumina bzw. Atemkapazitäten, mit einem Atembeutel, der über ein erstes Ventilsystem mittels Atemrohr an den Probanden und über ein weiteres Ventilsystem an Testgasbehälter anschliessbar ist, und einer Gasanalysevorrichtung zur kontinuierlichen Messung der Testgaskonzentration im Atembeutel, wobei das von der Testgaskonzentration abhängige Messsignal unmittelbar als Lungenfunktionsmessgrösse darstellbar ist.

Zur Bestimmung von funktionellen Atemvolumina und/oder Atemkapazitäten wurden bisher beispielsweise Stickstoff-Auswaschungen der Lunge durchgeführt. Hierzu sind Volumenmessungen, beispielsweise mit einem Spirometer, einerseits und Konzentrationsmessungen des Atemgases andererseits notwendig. Aus der DE-B2-2 541 691 ist ein Gerät zur Durchführung derartiger Stickstoff-Auswaschungen vorbekannt, bei dem die Volumenmessung vereinfacht ist und nur noch eine genaue Stickstoffkonzentrationsmessung durchgeführt werden muss. Diese Konzentrationsmessung erfolgt dort diskontinuierlich nach Auswaschen des Stickstoffs mittels dem Atembeutel entnommener Teilproben durch chemische Analysemethoden.

Neben den speziellen Stickstoff-Auswaschungen der Lunge sind für den gleichen Messzweck auch Einwaschungen von definierten Testgasmengen, vorzugsweise Edelgasen, in die Lunge bekannt. Messgrössen sind dabei im wesentlichen Anfangs- und Endkonzentration des Testgases. Es ist also wiederum eine möglichst genaue Konzentrationsmessung erwünscht, wozu entsprechende Gasanalysevorrichtungen notwendig sind. Bei bekannten Geräten erfolgt die Konzentrationsmessung durch Bestimmung der Wärmeleitfähigkeit, die aber bei Verwendung von Edelgasen ziemlich ungenau ist, so dass die Lungenfunktionsanalyse unbefriedigend bleibt.

Ein Gerät der eingangs genannten Art ist aus der US-A-3 527 205 bekannt. Bei diesem Gerät ist eine Konzentrationsmessung mittels Gasanalysevorrichtung durch kontinuierliche Bestimmung des Gemisches von Testgas und Atemluft im Atembeutel möglich, wozu die Gasanalysevorrichtung als «By-pass»-Leitung dem Atemkreislauf parallel geschaltet ist. Das Messsignal kann in diesem Fall entsprechend der vorliegenden Testgaskonzentration unmittelbar als Lungenfunktionsmessgrösse auf dem Messgerät dargestellt werden. Daneben wird in der US-A-3 659 590 ein ähnliches Gerät beschrieben, bei dem die Gasanalysevorrichtung speziell auf dem Prinzip der Messung der Wärmeleitfähigkeit arbeitet. Dafür sind entsprechende Signalerfassungs- und Verarbeitungsmittel notwendig, wobei – wie bereits erwähnt – bei Verwendung von Edelgasen als Testgas die Messgenauigkeit unbefriedigend bleibt.

Bei allen vorbeschriebenen Geräten wird zur Atemvolumenbestimmung ein sogenanntes «bag-in-box»-System verwendet, d.h. der eigentliche Atembeutel befindet sich in einem gasmässig isolierten weiteren Kasten. Die Gasanalyse erfolgt immer ausserhalb dieses Systems.

Der Erfindung liegt daher die Aufgabe zugrunde, ein Gerät zur Lungenfunktionsanalyse zu schaffen, das einfach aufgebaut ist, keine komplizierten Gasanalysevorrichtungen aufweist und ohne «By-pass»-System auskommt, aber möglichst genaue Aussagen über die Lungenfunktion ermöglicht.

Die Aufgabe wird bei einem Gerät der eingangs genannten Art erfindungsgemäss dadurch gelöst, dass mittels eines separaten Anschlusses eine Messsonde in den Atembeutel einführbar ist, über welche dem Atembeutel eine definierte Wechselströmung bekannter Frequenz einprägbar ist, und dass die Testgaskonzentration durch den sich an der Sonde gegenüber Aussendruck ausbildenden Wechseldruck als gasdichteabhängiges Signal erfassbar ist.

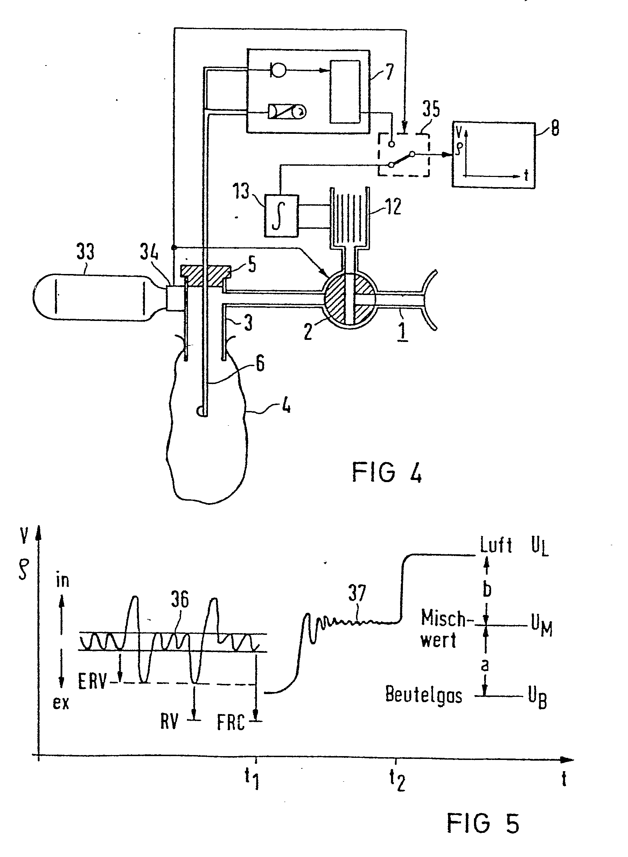

Bei einem Gerät nach der Erfindung wird also als Mass für die Gaskonzentration die Gasdichte kontinuierlich im Atembeutel gemessen und direkt auf einem Registriergerät angezeigt. Derartige Gasdichte-Kurven können schon unmittelbar zur Lungenfunktionsanalyse herangezogen werden. In Verbindung mit einer zusätzlichen Volumenmessung können daraus die Atemvolumina wie auch als besonders interessierende Messgrösse das Residualvolumen (RV) bestimmt werden. Ebenso kann die funktionelle Residualkapazität (FRC) bestimmt werden; der Zusammenhang zwischen diesen Grössen ergibt sich weiter unten aus Fig. 5.

Während bei bekannten Geräten für eine hinreichend genaue Konzentrationsmessung entweder diskontinuierliche chemische Methoden angewandt wurden, für kontinuierliche Messungen ein aufwendiges Massenspektrometer oder zumindest ein zusätzlich angeschaltetes «By-pass»-System notwendig waren, ist die bei dem erfindungsgemäss ausgebildeten Gerät verwendete Messsonde äusserst einfach aufgebaut. Vorzugsweise ist die Messsonde als Hohlrohr realisiert. Ein solches Hohlrohr ist vorteilhafterweise an jede beliebige Stelle im Atembeutel schiebbar.

Das so geschaffene Gerät zur Lungenfunktionsanalyse ist daher nach Aufbau und Betriebsweise äusserst einfach; ein Teil der Gerätekomponenten können auch für andere Messzwecke im Rahmen der Lungenfunktionsprüfung eingesetzt werden. Verwendet man als Testgasbehälter Einmal-Patronen mit definierter Testgasmenge bekannter Konzentration, aus denen eine bekannte Testgasmenge direkt in den Atembeutel geleitet wird, ergibt sich eine mobile Messeinrichtung ohne die bisher für den Transport störenden Gasflaschen.

Weitere Vorteile und Einzelheiten der Erfindung ergeben sich aus der nachfolgenden Figurenbeschreibung von verschiedenen Ausführungsbeispielen anhand der Zeichnung.

Es zeigen:

Fig. 1 schematisch einen Messaufbau zur Bestimmung der Einwaschzeit von Testgasen,

Fig. 2 schematisch einen Messaufbau zur Bestimmung des Residualvolumens (RV) bzw. der funktionellen Residualkapazität (FRC),

Fig. 3 einen gegenüber Fig. 2 abgewandelten Messaufbau zur vollständigen Testgas-Einwaschung,

Fig. 4 einen gegenüber Fig. 2 weiter abgewandelten, als mobile Einheit konzipierten Messaufbau und

Fig. 5 Registrierkurven zur Lungenfunktionsanalyse, die mit einem Gerät nach Fig. 2 bzw. 4 aufgenommen wurden.

In den Figuren sind identische Teile mit gleichen Bezugszeichen versehen.

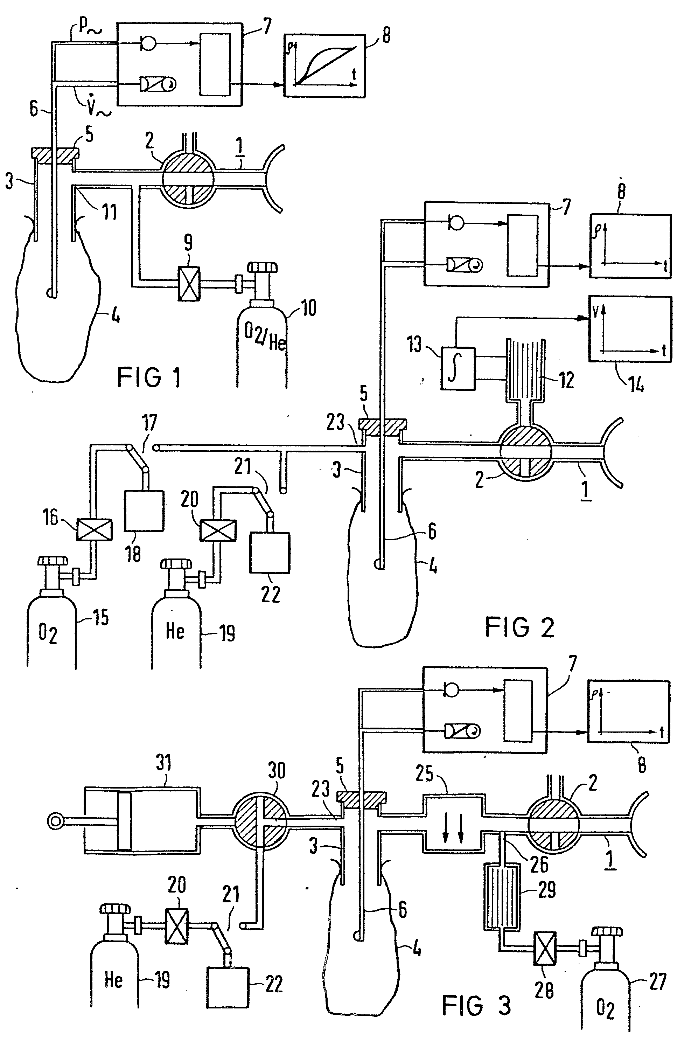

In der Fig. 1 ist mit 1 ein Atemrohr mit einem mundseitigen Anschluss für einen Probanden bezeichnet. Vor dem mundseitigen Ende des Atemrohres 1 befindet sich ein Dreiwegeventil 2, über das der Proband bei Bedarf Aussenluft (Raumluft) atmen kann. Das dem Probanden abgewandte Ende des Atemrohres ist als Winkelstück 3 ausgebildet. Am offenen Ende ist ein Atembeutel 4 angeordnet, wobei der dem offenen Ende gegenüberliegende Teil des Winkelstückes 3 als Anschluss 5 für eine in den Atembeutel 4 einführbare Messsonde 6 ausgebildet ist. Die Messsonde 6 besteht aus einem Hohlrohr zur Einprägung einer Wechselströmung $\dot{V}_\sim$ bekannter Grösse und Frequenz, bei der nahe am offenen Ende des Hohlrohres im Atembeutel 4 der Wechseldruck $p_\sim$ abgenommen wird. Strömungsleitung und Druckmessleitung der Messsonde 6 sind ausserhalb des Atembeutels 4 an ein Betriebsgerät 7 angeschlossen, das einerseits die definierte Wechselströmung erzeugt und andererseits die erfasste Wechseldruckamplitude als elektrisches Signal misst, elektronisch aufbereitet und als Analogsignal ausgibt. Dieses Analogsignal ist auf einem Registriergerät 8 als Zeitfunktion darstellbar. Über ein Reduzierventil 9 kann über ein Einlassventil 11 dem Atembeutel 4 Testgas aus einer Gasflasche 10 zugeführt werden. Das Einlassventil 11 ist am Atemrohr 1 oder unmittelbar am Anschluss 5 angeordnet.

Zur Durchführung der Lungenfunktionsanalyse wird bei geschlossenem Dreiwegeventil 2 über das Reduzierventil 9 eine beliebige Menge des Testgasgemisches aus der Gasflasche 10 in den Atembeutel 4 eingefüllt. Anschliessend atmet der Proband ca. 1 bis 3 Minuten in Hin- und Rückatmung in den Atembeutel 4, wobei das Gasdichtesignal kontinuierlich auf dem Registriergerät 8 aufgezeichnet wird. Bei Probanden mit normaler Lungenfunktion wird das Signal zunächst schnell ansteigen und nach ca. 1 Minute asymptotisch in eine Sättigung einlaufen. Bei pathologischen Verhältnissen dagegen steigt das gasdichte Signal langsam an; eine asymptotische Sättigung wird unter Umständen nicht erreicht. Es liegt eine Verteilungsstörung vor, die eine exakte Messung des Residualvolumens notwendig macht.

In der Fig. 2 bedeuten 1 wiederum ein Atemrohr mit Dreiwegeventil 2 zum mundseitigen Ende und Anschlussstutzen 3 und 5 am anderen Ende für einen Atembeutel 4 sowie eine darin einführbare Messsonde 6. Die Messsonde 6 ist wiederum an das Betriebsgerät 7 angeschlossen, von welchem das Messsignal auf das Registriergerät 8 gegeben wird. Am Dreiwegeventil 2 ist weiterhin ein Spirozeptor 12 zur Atemstrommessung angeschlossen, von dem über einen Integrator 13 das Atemvolumen V auf ein weiteres Registriergerät 14 zur Aufzeichnung gegeben wird. Statt des Spirozeptors 12 mit Integrator 13 kann auch ein geschlossenes Spirometer verwendet werden. Am Atembeutel 4 sind über ein Leitungssystem Testgasbehälter anschliessbar. Im einzelnen werden dafür Überdruckgasflaschen 15 und 19 über Reduzierventile 16 und 20 und pneumatische Schalter 17 und 21 mit Zwischenvolumina 18 und 22 verbunden, wobei nach Druckreduzierung auf etwa 3 bar jeweils definierte Volumina mit den Testgasen auffüllbar sind. Über die pneumatischen Umschalter 17 und 21 werden die Testgasvolumina über ein Einlassventil 23 in den Atembeutel 4 eingespeist. Zweckmässigerweise beinhaltet die erste Überdruckgasflasche reines Helium und die zweite reinen Sauerstoff, so dass sich im Atembeutel 4 gewünschte Testgasmengen definierter Testgaskonzentration leicht einstellen lassen.

Zur Bestimmung der FRC sind in diesem Fall neben den quantitativen Konzentrationsmesswerten auch das eingefüllte Volumen als Rechengrösse notwendig. Die Heliumbilanz ergibt folgende Beziehung:

$$(1) \qquad V_1 \cdot X_1 = (V_1 + V_0 + FRC) \cdot X_2$$

wobei $V_1$ das aus den Gasflaschen 15 bzw. 19 eingefüllte Testgasvolumen, $V_0$ das starre Systemvolumen (Totraum) und FRC die gesuchte Residualkapazität bedeuten. $X_1$ und $X_2$ sind die zugehörigen Heliumkonzentrationen.

Wird aus dem Atembeutel 4 Luft vom Probanden hin- und rückgeatmet, ist die relative Änderung der Gasdichte geringer als diejenige der Wärmeleitfähigkeit. Bei Hin- und Rückatmung eines durch den Heliumgehalt deutlich leichteren Testgasgemisches kann daher jede Dichteänderung des Gasgemisches im Atembeutel 4 unmittelbar als Änderung der Heliumkonzentration aufgefasst werden. Da man von einem streng linearen Zusammenhang zwischen der Dichte und der Signalspannung am Betriebsgerät 7 für die Messsonde 6 ausgehen kann, kann auf die absolute Bestimmung der Gaskonzentration verzichtet werden. Es genügt die Registrierung der Ausgangssignale des Betriebsgerätes 7 auf dem Registrierer 8 für das Beutelgas ($U_B$), das Mischgas ($U_M$) und für Normalluft ($U_L$). Aus Gleichung (1) folgt damit:

$$(2) \qquad FRC = V_1 \cdot \frac{U_M - U_B}{U_L - U_M} - V_0$$

Der Proband atmet zunächst über den Spirozeptor 12 Raumluft. Die Atemvolumenkurve wird am Registriergerät 14 aufgezeichnet. Nach Einfüllen des definierten Testgasvolumens $V_1$ mit einer Konzentration $X_1$ in den Atembeutel 4 wird der Proband bei einer der gewünschten Messgrösse entsprechenden Atemlage an den Atembeutel 4 angeschlossen. Nach relativ kurzer Rückatmungszeit (etwa 1 bis 5 Minuten) kann der Proband bei beliebiger Atemlage vom System getrennt werden. FRC ergibt sich dann unmittelbar aus Gleichung (2) mit den für $U_B$, $U_M$ und $U_L$ abgelesenen Werten.

In der Fig. 3 ist das Ausführungsbeispiel nach Fig. 2 in der Weise modifiziert, dass eine Hin- und Rückatmung in den Atembeutel 4 bis zur vollständigen Helium-Einwaschung in die Lunge durchführbar ist. Eine derartige vollständige Einwaschung kann bis zu 10 Minuten dauern. Bei solcher längerer Hin- und Rückatmung in den Atembeutel 4 ist eine $CO_2$-Absorption aus dem Atemgas sowie Sauerstoffnachlieferung für den Probanden notwendig. Zwischen dem Dreiwegehahn 2 und dem Atembeutel 4 ist dafür ein $CO_2$-Absorber 25 eingeschaltet. Über einen Gaseinlass 26 wird reiner Sauerstoff aus einer Gasflasche 27 mit Reduzierventil 28 und Strömungsmesser 29 in das Atemrohr 1 für den Probanden nachgeliefert.

Ein Spirometer zur Atemstrom- bzw. Atemvolumenmessung ist im Ausführungsbeispiel nach Fig. 3 nicht vorhanden. In diesem Fall ist auch lediglich eine Überdruckgasflasche 19 mit Reduzierventil 20 vorgesehen, mit dem über den pneumatischen Umschalter 21 und Normvolumenbehälter 22 reines Helium in den Atembeutel 4 eingefüllt wird. Vom Atembeutel 4 kann über ein Ventil 30 mit einer mechanischen Kolbenpumpe 31 das Mischgas abgezogen und so das Beutelvolumen am Abschluss einer Messung festgestellt werden.

Geht man von reinem Helium als Testgas aus, so ergibt sich entsprechend Gleichung (1) die Heliumbilanz:

$$(3) \qquad V_{He} = (V_2 + V_o + FRC) \cdot X_2$$

wobei neben den bereits bekannten Grössen $V_{He}$ das Volumen des reinen Helium-Gases und $V_2$ das Volumen im Atembeutel nach beendigter Messung (also vollständiger Helium-Einwaschung) bedeuten.

Zur Durchführung der Messung wird ein definiertes Heliumvolumen $V_{He}$ in den Atembeutel 4 eingefüllt, ein beliebiger Sauerstoff- oder Luftanteil hinzugefüllt und der Proband nach Exspiration an den Atembeutel 4 angeschlossen. Der Proband atmet nun so lange, bis auf dem Schreiber 8 die Enddichte asymptotisch erreicht ist. Während dieser möglicherweise längeren Rückatmungszeit wird in der Exspirationsphase das Kohlendioxid im Absorber 25 absorbiert. Zur Atmung benötigter Sauerstoff wird in geeigneter Weise aus der Gasflasche 27 zugeführt.

Der Messaufbau nach Fig. 4 ist gegenüber dem Aufbau in Fig. 2 in der Weise modifiziert, dass der Schreiber 8 über einen Wechselschalter 35 alternativ an den Konzentrationssignalgeber oder den Volumensignalgeber angeschlossen werden kann. Statt der Überdruckgasbehälter werden nun an den Atembeutel 4 Einweggaspatronen 33 angeschlossen. Solche Gaspatronen 33 beinhalten beispielsweise 1 Liter eines Testgases bei 5 bar, geben also 5 Liter bei Atmosphärendruck ab. Diese Menge ist für eine Messung geeignet. Mit Anschluss der Patrone 33 an den als Einlassventil 34 ausgebildeten Anschluss werden über eine elektrische Rückkopplung gleichzeitig das Dreiwegeventil 2 im Atemrohr und der Schalter 35 umgesteuert. Damit ist der gesamte Messvorgang weitgehend automatisiert.

In der Fig. 5 bedeuten 36 eine Atemvolumenkurve eines Probanden und 37 eine Dichtemesskurve entsprechend dem Konzentrationsverlauf im Atembeutel 4 bei einer Messung. Die Atemvolumenkurve 36 gibt eine Anzahl von in- und exspiratorischen Atemzügen wieder, wobei der Proband mit normalem Atemvolumen (AV) atmet. Die signifikanten Lungenfunktionsmessgrössen sind das Residualvolumen (RV) und die funktionelle Residualkapazität (FRC). Dabei unterscheiden sich beide Grössen um das exspiratorische Reservevolumen (ERV). Je nachdem, in welcher Atemlage mit dem erfindungsgemässen Gerät die Messung begonnen wird, wird das dementsprechende Atemvolumen bzw. die Atemkapazität als Lungenfunktionsmesswert bestimmt. Es kann vorteilhaft sein, bei einem Messaufbau nach Fig. 1 bis 4 einem Spirozeptor als Atemstromsignalgeber einen Mikroprozessor zuzuordnen, durch den die Messung in einer bestimmten erwünschten Atemlage gesteuert wird. Bei der Messwertausgabe kann dann unmittelbar in RV und FRC unterschieden werden.

In Fig. 5 ist der Schalter 35 nach Fig. 4 zunächst auf Atemvolumenregistrierung geschaltet. Mit Anschluss der Testgaspatrone 33 an den Atembeutel 4 wird bei der erwünschten Atemlage auf Beutelatmung und zugleich der Registrierer 8 auf Dichteanzeige umgeschaltet. Das Dichtesignal steigt zunächst vom reinen Testgaswert $U_B$ schnell an, um sich bei laufender Hin- und Rückatmung an den asymptotischen Grenzwert $U_M$ einzupendeln. Aus der Signalstruktur sind die einzelnen Atemzüge noch erkennbar. Ist der Grenzwert erreicht, kann der flexible Atembeutel 4 vom Rohransatz 3 abgezogen werden. Das an der Messsonde 6 abgenommene Dichtesignal stellt sich dann sofort auf Luftwert $U_L$ ein. In der Registrierkurve 37 sind die Differenzen der relativen Signalwerte mit

$$U_M - U_B = a$$
und
$$U_L - U_M = b$$

bezeichnet, so dass sich Gleichung (2) entsprechend schreiben lässt

$$(2a) \qquad FRC = V_1 \cdot \frac{a}{b} - V_o$$

Da $V_o$ möglichst klein gehalten wird und für einen gewählten Messaufbau konstant ist, kann diese Grösse vernachlässigt bzw. eingeeicht werden. Aus dem unmittelbar aus der Registrierkurve 37 ablesbaren Verhältnis a/b ergibt sich dann der gesuchte Messwert.

Mit der an den Ausführungsbeispielen beschriebenen Erfindung ist die Bestimmung von Lungenfunktionsmessgrössen in einfachster Weise möglich. Insbesondere durch Verwendung des mit definierter Wechselströmung beaufschlagten Hohlrohrs als in den Atembeutel einführbare Gasdichtemesssonde und Messung der Wechseldruckamplitude am Hohlrohr sind aufwendige Konzentrationsmessungen von Testgaskomponenten überflüssig. Speziell für Helium als Testgaskomponente ergibt sich eine genügende Empfindlichkeit des Gasdichtesignals als Konzentrationsmass dann, wenn der Wechseldruck nahe am offenen Ende des Hohlrohrs abgenommen wird. Gute Ergebnisse wurden bei Verwendung von Hohlrohren mit einem Durchmesser zwischen 2 und 5 mm, einer Wechselströmung mit einer Wechselströmungsamplitude zwischen 10 und 50 ml/s und einer Frequenz zwischen 10 und 100 Hz erzielt, wobei der Abstand der Druckmessstelle vom Hohlrohrende geringer als der innere Durchmesser des Hohlrohrs gewählt wurde.

**Patentansprüche**

1. Gerät zur Lungenfunktionsanalyse, insbesondere zur Messung von funktionellen Atemvolumina bzw. Atemkapazitäten, mit einem Atembeutel (4), der über ein erstes Ventilsystem (2) mittels Atemrohr (1) an den Probanden und über ein weiteres Ventilsystem (11, 17, 21, 23, 30, 34) an Testgasbehälter (10, 15, 18, 19, 22, 33) anschliessbar ist, und einer Gasanalysevorrichtung zur kontinuierlichen Messung der Testgaskonzentration ($X_i$) im Atembeutel (4), wobei das von der Testgaskonzentration ($X_i$) abhängige Messsignal unmittelbar als Lungenfunktionsmessgrösse (RV, FRC) darstellbar ist, dadurch gekennzeichnet, dass mittels eines separaten Anschlusses (5) eine Messsonde (6) in den Atembeutel (4) einführbar ist, über welche dem Atembeutel (4) eine definierte Wechselströmung ($\dot{V}_\sim$) bekannter Frequenz einprägbar ist, und dass die Testgaskonzentration ($X_i$) durch den sich an der Sonde (6) gegenüber Aussendruck ausbildenden Wechseldruck ($p_\sim$) als gasdichteabhängiges Signal ($U[\rho]$) erfassbar ist.

2. Gerät nach Anspruch 1, dadurch gekennzeichnet, dass die Messsonde (6) aus einem Hohlrohr besteht.

3. Gerät nach Anspruch 1 oder 2, dadurch gekennzeichnet, dass das gasdichteabhängige Signal ($U[\rho]$) durch einen auf die Frequenz der Wechselströmung ($\dot{V}_\sim$) abgestimmten Druckmesser gemessen wird.

4. Gerät nach einem der Ansprüche 1 bis 3, dadurch gekennzeichnet, dass Wechselströmungserzeuger und Druckmesser zusammen in einem auf die verwendete Messsonde (6) abgestimmten Betriebsgerät (7) angeordnet sind, das die Wechseldruckamplitude als elektrisches Analogsignal ausgibt.

5. Gerät nach einem der Ansprüche 1 bis 4, dadurch gekennzeichnet, dass zur quantitativen Bestimmung der Lungenfunktionsmessgrösse (RV, FRC) das Analogsignal ($U[\rho]$) auf einem Registriergerät (8) dargestellt wird, wozu die relativen Signalwerte für Testgas ($U_B$), für Mischluft ($U_M$) und Raumluft ($U_L$) zwecks Verrechnung erfassbar sind.

6. Gerät nach Anspruch 1, dadurch gekennzeichnet, dass an das zweite Ventilsystem (11, 17, 21, 23, 30, 34) mehrere Testgasbehälter (15, 19) mit unterschiedlichen Testgasen angeschaltet sind.

7. Gerät nach Anspruch 6, dadurch gekennzeichnet, dass die Testgasbehälter (15, 19) Überdruckgasbehälter sind, denen Reduzierventile (16, 20) und Zwischenbehälter (18, 22) nachgeschaltet sind, wobei über pneumatische Umschalter (17, 21) die Zwischenbehälter (18, 22) jeweils zuerst mit dem Testgasbehälter (15, 19) und anschliessend mit dem Atembeutel (4) verbindbar sind.

8. Gerät nach einem der Ansprüche 1 bis 5, dadurch gekennzeichnet, dass für längere Hin- und Rückatmung zur vollständigen Testgaseinwaschung dem Atemrohr (1) Mittel (25, 27, 29) zur $CO_2$-Absorption aus dem Atemgas und zur Sauerstoffnachlieferung in das Atemgas zugeordnet sind.

9. Gerät nach einem der Ansprüche 1 bis 5, dadurch gekennzeichnet, dass dem Atembeutel (4) Mittel zur Messung des Endvolumens ($V_2$) des Mischgases im Atembeutel (4) zugeordnet sind.

10. Gerät nach einem der Ansprüche 1 bis 5, dadurch gekennzeichnet, dass der Testgasbehälter eine Einwegpatrone (33) ist, welche mit einer definierten Testgasmenge bekannter Konzentration und Überdruck vorgefüllt ist und direkt an das zweite Ventilsystem (34) zum Einfüllen des Testgases in den Atembeutel (4) angeschlossen wird.

11. Gerät nach einem der Ansprüche 1 bis 5, dadurch gekennzeichnet, dass an das erste Ventilsystem (2) Atemvolumenmesser (12, 13) anschliessbar sind, von denen Atemvolumenkurven auf ein weiteres Registriergerät (14) gebbar sind.

12. Gerät nach Anspruch 11, dadurch gekennzeichnet, dass Atemvolumenkurve (V) und Gasdichtesignal ($U[\rho]$) mittels eines Schalters (35) wechselweise auf das gleiche Registriergerät (8) gebbar sind, wobei nach Einspeisen des Testgasvolumens in den Atembeutel (4) automatisch von Volumenregistrierung auf Dichteregistrierung umgeschaltet wird.

13. Gerät nach einem der vorhergehenden Ansprüche, dadurch gekennzeichnet, dass die Anschaltung des Probanden an den mit Testgas gefüllten Atembeutel (4) und Registrierung des Gasdichtesignals ($U[\rho]$) bei einer vorwählbaren Atemlage des Probanden erfolgt, wobei sich abhängig von der gewählten Atemlage der entsprechende Lungenfunktionsmesswert (RV, FRC) ergibt.

14. Gerät nach Anspruch 1 oder 13, dadurch gekennzeichnet, dass die Ermittlung und Anzeige der der Atemlage entsprechenden Lungenfunktionsmessgrösse (RV, FRC) durch einen Mikroprozessor gesteuert erfolgt.

## Claims

1. Lung-function analysis apparatus, in particular for the measurement of functional breath volumetric quantities and breathing capacities, comprising a breath bag (4) connected via a first valve system (2) to a breathing tube (1) for the patient to be tested, and via a further valve system (11, 17, 21, 23, 30, 34) to test gas containers (10, 15, 18, 19, 22, 23), a gas analysis device being provided for the continuous measurement of the test gas concentration $(X_i)$ in the breath bag (4), where the measurement signal which is dependent upon the test gas concentration $(X_i)$ may be directly represented as a lung function measurement value (RV, FRC), characterised in that via a separate connection (5) a measurement probe (6) may be introduced into the breath bag (4) via which probe a determinate alternating flow $(\dot{V}\sim)$ of known frequency may be impressed upon the breath bag (4), and that the test gas concentration $(X_i)$ may be detected in the form of a gas-density-dependent signal $(U[\rho])$ by means of the alternating pressure $(p\sim)$ which forms on the probe (6) relative to the outer pressure.

2. Apparatus as claimed in Claim 1, characterised in that the measurement probe (6) consists of a hollow tube.

3. Apparatus as claimed in Claim 1 or 2, characterised in that the gas-density-dependent signal $(U[\rho])$ is measured by a pressure meter which is tuned to the frequency of the alternating flow $(\dot{V}\sim)$.

4. Apparatus as claimed in one of the Claims 1 to 3, characterised in that the alternating flow generator and pressure meter are arranged together in an operating device (7) which is tuned to the measurement probe (6) being used and which emits the alternating pressure amplitude as an electrical analogue signal.

5. Apparatus as claimed in one of the Claims 1 to 4, characterised in that for the quantitative determination of the lung function measurement valve (RV, FRC) the analogue signal $(U[\rho])$ is represented on a recording device (8), for the relative signal values for test gas $(U_B)$, for mixed air $(U_M)$ and spatial air $(U_L)$ to be detected for purposes of calculation.

6. Apparatus as claimed in Claim 1, characterised in that the second valve system (11, 17, 21, 23, 30, 34) is connected to a plurality of test gas containers (15, 19) containing different test gases.

7. Apparatus as claimed in Claim 6, characterised in that the test gas containers (15, 19) are excess pressure gas containers followed by reducing valves (16, 20) and intermediate containers (18, 22), the intermediate containers (18, 22) being connected via pneumatic change-over switches (17, 21) firstly to the test gas container (15, 19) and subsequently to the breath bag (4).

8. Apparatus as claimed in one of the Claims 1 to 5, characterised in that for a long period of breathing in and out for complete washing of the test gas the breathing tube (1) is assigned means (25, 27, 29) for $CO_2$ absorption from the breath gas and for the supply of oxygen to the breath gas.

9. Apparatus as claimed in one of the Claims 1 to 5, characterised in that the breath bag (4) is assigned means to measure the final volume $(V_2)$ of the mixed gas in the breath bag (4).

10. Apparatus as claimed in one of the Claims 1 to 5, characterised in that the test gas container is a one-way cartridge (33) which is pre-filled with a determinate quantity of test gas of known concentration and excess pressure, and is directly connected to the second valve system (34) in order to introduce the test gas into the breath bag (4).

11. Apparatus as claimed in one of the Claims 1 to 5, characterised in that the first valve system (2) can be connected to breath volumetric quantity meters (12, 13), from which curves depicting breath volumetric quantities may be fed to a further recording device (14).

12. Apparatus as claimed in Claim 11, characterised in that the breath volumetric quantity curve (V) and gas density signal $(U[\rho])$ may be fed alternately by means of a switch (35) to the same recording device (8), where, following the feed-in of the test gas volumetric quantity into the breath bag (4), an automatic switch-over takes place from volumetric quantity recording to density recording.

13. Apparatus as claimed in one of the preceding Claims, characterised in that connection to the patient who is to be tested by the breath bag (4) filled with test gas, and the recording of the gas density signal $(U[\rho])$ takes place in a preselectable breathing state of the patient to be tested, and in dependence upon the selected breathing condition the appropriate lung function measurement value (RV, FRC) is produced.

14. Apparatus as claimed in Claim 1 or 13, characterised in that the determination and display of the lung function measurement value (RV, FRC) corresponding to the breathing condition take place under the control of a micro-processor.

## Revendications

1. Appareil pour l'analyse de la fonction pulmonaire, notamment pour la mesure de volumes respiratoires fonctionnels ou capacités respiratoires, qui comporte un sac respiratoire (4), qui peut être relié aux patients au moyen d'un tube respiratoire (1), par l'intermédiaire d'un premier système de soupape (2), et à des réservoirs de gaz d'essai (10, 15, 18, 19, 22, 33), par l'intermédiaire d'un autre système de soupapes (11, 17, 21, 23, 30, 34), ainsi qu'un dispositif d'analyse de gaz pour mesurer de façon continue la concentration $(X_i)$ du gaz d'essai dans le sac respiratoire (4), le signal de mesure, qui dépend de la concentration $(X_i)$ du gaz d'essai, pouvant être représenté directement en tant que grandeur de mesure (RV, FRC) de la fonction pulmonaire, caractérisé par le fait qu'une sonde de mesure (6), qui permet d'envoyer un écoulement alternatif défini $(\dot{V}\sim)$ de fréquence connue au sac respiratoire (4), peut être introduite dans le sac respiratoire (4) au moyen d'un raccord distinct (5), et que la concentration du gaz d'essai ($X_l$) peut être déterminée sous forme d'un signal fonction de la densité du gaz, par la pression alternative ($p_\sim$) s'établissant par rapport à la pression extérieure au niveau de la sonde (6).

2. Appareil suivant la revendication 1, caractérisé par le fait que la sonde de mesure (6) est constituée par un tube creux.

3. Appareil suivant l'une des revendications 1 ou 2, caractérisé par le fait que le signal qui dépend de la densité du gaz ($U[\rho]$) est mesuré au moyen d'un manomètre accordé sur la fréquence de l'écoulement alternatif ($\dot{V}_\sim$).

4. Appareil suivant l'une des revendications 1 à 3, caractérisé par le fait que les générateurs d'écoulement alternatif et le manomètre sont disposés ensemble dans un appareil de service (7) qui est accordé sur la sonde de mesure utilisée (6), et qui délivre l'amplitude de la pression alternative sous la forme d'un signal analogique électrique.

5. Appareil suivant l'une des revendications 1 à 4, caractérisé par le fait que pour déterminer quantitativement les grandeurs de mesure (RV, FRC) de la fonction pulmonaire, le signal analogue ($U[\rho]$) est représenté sur un appareil d'enregistrement (8), des valeurs de signaux relatives pour le gaz d'essai ($U_B$), le mélange de gaz ($U_M$) et l'air ambiant ($U_L$) pouvant être détectées à cet effet pour être prises en compte.

6. Appareil suivant la revendication 1, caractérisé par le fait que plusieurs réservoirs de gaz d'essai (15, 19), qui contiennent des gaz d'essai différents, sont raccordés au second système de soupapes (11, 17, 21, 23, 30, 34).

7. Appareil suivant la revendication 1, caractérisé par le fait que les réservoirs de gaz d'essai (15, 19) sont des réservoirs de gaz sous pression en aval desquels sont montés des détendeurs (16, 20) et des réservoirs auxiliaires (18, 22), les réservoirs auxiliaires (18, 22) pouvant être reliés tout d'abord avec le réservoir de gaz sous pression (15, 19) puis avec le sac respiratoire (4), par l'intermédiaire de commutateurs pneumatiques (17, 21).

8. Appareil suivant l'une des revendications 1 à 5, caractérisé par le fait que pour une respiration complète plus longue, afin que tout le gaz d'essai pénètre dans le tube respiratoire (1), on prévoit des moyens (25, 27, 29) pour absorber le $CO_2$ et pour introduire de l'oxygène dans le gaz respiré.

9. Appareil suivant l'une des revendications 1 à 5, caractérisé par le fait que des moyens sont associés au sac respiratoire (4) pour la mesure du volume final ($V_2$) du mélange de gaz dans le sac respiratoire (4).

10. Appareil suivant l'une des revendications 1 à 5, caractérisé par le fait que le réservoir de gaz d'essai est une cartouche à jeter (33), qui est préremplie d'une quantité définie de gaz d'essai de concentration et de pression connues, et qui est raccordée directement au second système de soupape (34) pour introduire le gaz d'essai dans le sac (4).

11. Appareil suivant l'une des revendications 1 à 5, caractérisé par le fait qu'au premier système de soupapes (2) peuvent être raccordés des dispositifs de mesure du volume respiratoire (12, 13) dont les courbes de volumes respiratoires peuvent être fournies à un autre appareil d'enregistrement (14).

12. Appareil suivant la revendication 11, caractérisé par le fait que la courbe de volume respiratoire (V) et le signal de densité de gaz ($U[\rho]$) peuvent être appliqués alternativement au même appareil d'enregistrement (8), au moyen d'un commutateur (35), ce dispositif passant automatiquement de l'enregistrement du volume à l'enregistrement de densité après introduction du volume de gaz d'essai dans le sac respiratoire (4).

13. Appareil suivant l'une des revendications précédentes, caractérisé par le fait que le raccordement du patient au sac respiratoire (4) rempli de gaz d'essai et l'enregistrement du signal de densité et de gaz ($U[\rho]$) ont lieu pour une phase de respiration du patient qui peut être choisie, la valeur de mesure correspondante de la fonction respiratoire (RV, FRC) étant obtenue en fonction de la phase de respiration choisie.

14. Appareil suivant l'une des revendications 1 ou 13, caractérisé par le fait que la détermination et l'affichage de la grandeur de mesure de la fonction respiratoire (RV, FRC) qui correspond à la phase de respiration ont lieu sous la commande d'un microprocesseur.

0 017 162

FIG 1

FIG 2

FIG 3

9

FIG 4

FIG 5